# EUROPEAN PATENT APPLICATION

(11) **EP 4 663 764 A1**
(43) Date of publication of application: **17.12.2025**
(21) Application number: 24181142.1
(22) Date of filing: 10.06.2024
(51) Int. Cl.: C12P 7/40, C12P 7/52, C12P 7/54, C12P 7/625

(54) **A FERMENTATION PRODUCT AND A METHOD FOR PRODUCING A FERMENTATION PRODUCT**

(71) Applicant: NG Nordic Finland Oy, 11120 Riihimäki (FI)
(72) Inventor: PAWAR, Sudhanshu, 16968 Solna (SE); HUUSELA, Martina, 02150 Espoo (FI)
(74) Representative: Laine IP Oy

(57) **Abstract**

According to an example aspect of the present invention, there is provided a method comprising the steps of providing a microbial biomass comprising at least one microorganism expressing the enzymes of the Wood-Ljungdahl pathway; growing the microbial biomass under anaerobic conditions comprising carbon dioxide and hydrogen gas to form an enriched microbial biomass; producing a fermentation product comprising at least one short-chain fatty acid by using the enriched microbial biomass; wherein the producing step is performed at a temperature below 18°C.

## Description

### FIELD

The present disclosure belongs to the field of bioproducts. More specifically, the present disclosure belongs to the field of microbial bioproducts such as biopolymers.

### BACKGROUND

The need for plastics is expected to increase to 1100 Mt by 2050 and is expected to contribute to 19% of the world's greenhouse gas emission. Even when 63% of this plastic is expected to be recycled, there are limitations in current recycling method resulting in residual plastics waste that is either too complex or too hazardous to be recycled with current methodologies. Today 90% of the plastics is produced from fossil sources. Only 9% is recycled due to the challenges with chemical (pyrolysis) and mechanical recycling. Thus, the waste is mostly landfilled while part is incinerated with energy recovery.

Biodegradable polymers such as polyhydroxyalkanoates (PHA) can be produced by microbial fermentation. For example, polyhydroxybutyrate (PHB) can be produced from methane using preferable mixed cultures with cell debris recirculation. However, such PHB is known to be brittle, limiting its usability. In addition, while PHAs can be produced from various substrates, they are mostly produced from starch or lipids. Thus, the production of PHAs typically competes for the same raw material as food production, which has ethical challenges.

Production of PHAs can be dependent on the presence of volatile fatty acids. Especially volatile fatty acids that have longer carbon chain are advantageous for the properties of PHAs. However, addition of volatile fatty acids may increase substantially the price of produced PHA and thus methods for VFA production are sought after.

### SUMMARY OF THE INVENTION

The invention is defined by the features of the independent claims. Some specific embodiments are defined in the dependent claims.

According to a first aspect of the present invention, there is provided a method comprising the steps of: providing a microbial biomass comprising at least one microorganism expressing the enzymes of the Wood-Ljungdahl pathway; growing the microbial biomass under anaerobic conditions comprising carbon dioxide and hydrogen gas to form an enriched microbial biomass; producing a fermentation product comprising at least one short-chain fatty acid by using the enriched microbial biomass; wherein the producing step is performed at a temperature below 18°C.

Various embodiments of the first aspect may comprise at least one feature from the following bulleted list:
- said temperature during the producing step is below 15°C, such as below 12 °C.
- the temperature during at least some period of the growing step is below 18 °C, such as below 15 °C.
- the fermentation product comprises at least 40 g/L of short-chain fatty acids calculated from a liquid portion of the fermentation product.
- the short-chain fatty acid is selected from acetic acid, propionic acid, butyric acid, isobutyric acid, isovaleric acid, valeric acid, hexanoic acid or combinations thereof.
- the producing step is performed under anaerobic conditions comprising carbon dioxide and hydrogen gas.
- the pressure during the growing step and/or the producing step is between 1 to 12 bar.
- the pH during the growing step and/or the producing step is below 6.0.
- the microbial biomass is obtained from a sludge, for example from sludge from methane production or waste water treatment.
- the carbon dioxide is used as the only carbon source for the microbial biomass and/or for the enriched microbial biomass.
- the fermentation product comprises at least 1 %, such as at least 5 %, of valeric acid, isovaleric acid, hexanoic acid or combinations thereof of the total amount of produced short-chain fatty acids.
- the fermentation product is contacted with a second microbial culture to form at least one polyhydroxyalkanoate.
- the at least one polyhydroxyalkanoate is formed from the polymerization of 3-hydroxyvalerate, 3-hydroxybutyrate, 3-hydroxyhexanoate, 3-hydroxypropionate, 3-hydroxyoctanoate or combinations thereof.

According to a second aspect of the present invention, there is provided a fermentation product obtainable by the method according to the first aspect of the invention.

Various embodiments of the second aspect may comprise that at least 1 %, such as at least 5 %, of valeric acid, isovaleric acid, and/or hexanoic acid calculated from the weight of the liquid portion of the fermentation product.

### Advantages of the invention

An advantage of the invention is that it may enable production of short-chain fatty acids from waste materials.

Another advantage of the invention is that it allows affecting the amount and/or distribution of short-chain fatty acids produced during fermentation.

Another advantage of the invention is that it may provide a method for producing valeric acid, isovaleric acid and/or hexanoic acid from carbon dioxide.

Another advantage of the invention is that it may reduced the cost of producing biopolymers, such as polyhydroxyalkanoates.

### EMBODIMENTS

In the present context, the term "culture" refers to a population of unicellular or multicellular microorganisms in a medium, such as a growth or fermentation medium.

In the present context, the term "liquid portion of the fermentation product" refers to a portion that is obtained when the microbial biomass is separated and removed from the fermentation product. Microbial biomass may be separated and removed for example by centrifugation or by filtration. A person skilled in the art is well aware of methods suitable for microbial biomass separation and removal. For example, centrifugation may be performed for example at 5 000 - 6 000 x g for 5 to 10 minutes.

The present disclosure provides a method in which a microbial biomass is utilized in the production of bioproducts. Specifically, the method provides a method that allows formation of short-chain fatty acids by using a microbial culture while the production of methane is suppressed.

The present disclosure provides a method comprising the steps of: providing a microbial biomass comprising at least one microorganism expressing the enzymes of the Wood-Ljungdahl pathway; enriching the microbial biomass under anaerobic conditions comprising carbon dioxide and hydrogen gas to form an enriched microbial biomass; and producing at least one short-chain fatty acid using the enriched microbial biomass. The producing step is performed at a temperature below 18°C.

The low production temperature reduces growth of methane producing microorganisms, such as methanogens and/or methanogenic archaea. The term "methanogens" refers herein to microorganisms that produce methane as a metabolic product in hypoxic conditions. Specifically, the term "methanogens" refers herein to hydrogenotrophs that use carbon dioxide (CO₂) as a carbon source, and hydrogen as a reducing agent to form methane. These methane producing microorganisms may use carbon dioxide and hydrogen gas as well as short-chain fatty acids for the production of methane. Microorganisms producing methane are usually the dominant hydrogen utilizing microorganisms (hydrogenotrophs) in many environments. This may be because these microbes may have a lower threshold for H₂ than acetogens. In addition, the energy yield from the conversion of CO₂ and H₂ to methane is greater than that for conversion to acetate. Thus, a microbial biomass comprising methane producing microorganisms typically produce methane unless methane producing microorganisms are inhibited.

The Wood-Ljungdahl pathway, also known as reductive acetyl-coenzyme A (acetyl-CoA) pathway, allows microorganisms to use hydrogen (H₂) as an electron donor and carbon dioxide (CO₂) as an electron acceptor. Carbon dioxide is used as a carbon source by these microbes. The Wood-Ljungdhal pathway is characterized by the use of hydrogen as an electron donor and carbon dioxide as an electron acceptor to produce acetyl-CoA as the final product. The enzymes of Wood-Ljungdhal pathway attaches two carbon units derived from carbon dioxide directly to each other rather than adding carbon dioxide to an already formed carbon chain. Two specific enzymes are required for the pathway: carbon monoxide dehydrogenase and acetyl-CoA synthase. The former catalyses the reduction of CO₂ and the latter combines the resulting CO with a methyl group, which has also been formed from CO₂, to give acetyl-CoA. In addition to these two specific enzymes, many other enzymes are required for the pathway to be functional. Not to be confused with the Wood-Ljungdahl pathway, an evolutionarily related but biochemically distinct pathway named the Wolfe Cycle occurs exclusively in some methanogenic archaea called methanogens. In these anaerobic archaea, the Wolfe Cycle functions as a methanogenesis pathway to reduce CO₂ into methane (CH₄) with electron donors such as hydrogen (H₂) and formate (HCOO-).

Microorganisms expressing the enzymes of the Wood-Ljungdahl pathway comprise for example acetogens, which typically are obligately anaerobic bacteria. The term "acetogens" refers herein to microorganisms that produce acetate as an end product of anaerobic respiration or fermentation. In some examples, the microorganisms expressing the enzymes of the Wood-Ljungdahl pathway comprise or consists of homoacetogens. Homoacetogens may produce acetyl-CoA, and from that, in most cases, acetate as the end product, from two molecules of carbon dioxide (CO₂) and four molecules of molecular hydrogen (H₂). Examples of microorganisms expressing the enzymes of the Wood-Ljungdahl pathway comprise bacteria belonging to the genus Psuedomonas, Clostridium, Moorella, Citrobacter, Acetobacter, Cupriavidus, Calderihabitans, Carboxydothermus, and Defluviitoga.

Microorganisms expressing the enzymes of the Wood-Ljungdahl pathway may produce short-chain fatty acids. The term "short-chain fatty acids" refers herein to a group of fatty acids having two to six carbon atoms. These fatty acids are sometimes referred to as volatile fatty acids (VFA) because they can be separated by steam distillation at atmospheric pressure. Examples of short-chain fatty acids include acetic acid, propionic acid, butyric acid, isobutyric acid, isovaleric acid, valeric acid, and/or hexanoic acid. In the present context, the term "short-chain fatty acid" refers also to salts of short-chain fatty acids. Examples of salts of short-chain fatty acids include acetate, propionate, butyrate, valerate, isovalerate and succinate. In some embodiments, the short-chain fatty acid is selected from valeric acid, isovaleric acid, hexanoic acid or combinations thereof.

In some examples, the fermentation product comprises fatty acids having two to seven carbon atoms. In these examples, the fermentation product thus may comprise heptanoic acid in addition to short-chain fatty acids. For example, the fermentation product may comprise acetic acid, propionic acid, butyric acid, isobutyric acid, isovaleric acid, valeric acid, hexanoic acid and/or heptanoic acid.

In some embodiments, the microbial biomass may be obtained from a sludge, for example from a sludge that is produced during methane production or waste water treatment. Sludge may in some examples be collected in several different sites, such as separate fermenters, and combined in to one sludge. Sludge comprises many types of microorganisms forming a microbial community. The exact composition of the microbial community may be governed by many external/environmental factors, such as the temperature, pH, available nutrients, and availability of oxygen, as well as interactions of the microorganisms present in the community with each other. In some examples, the sludge may be pre-treated before it is provided to the method disclosed herein. Pre-treatment may be done so that the organic content comprised in the sludge is completely oxidised by the microorganisms within the sludge. This reduces or removes the carry-over of such organic content to the enrichment process. For example, the pre-treatment may result in all or at least some of the possible carbon sources within the sludge to be utilized/exhausted prior to the enrichment process. The pre-treatment may comprise for example incubating the sludge at an elevated temperature, for example at 25 to 35 °C, such as at about 30 °C, prior to providing the sludge to the disclosed method. The sludge may be incubated at an elevated temperature for example for at least 1 day, such as at least 5 days, for example about 5 to 7 days. In some examples, the sludge may be incubated with an amount of water under anaerobic conditions. Also in such examples, the temperature may be elevated as discussed above.

The provided microbial biomass is grown under anaerobic conditions comprising carbon dioxide and hydrogen gas to form an enriched microbial biomass. The term "enriched" refers here to a process in which the microbial biomass is grown under specific conditions in order to affect the composition of the microbial community within the microbial biomass. During the growing step, the microbial community within the microbial biomass changes so that some microorganism ceases to exist or their amount/number is reduced and others begin to thrive. Which microorganisms are enriched, is at least partly governed by the conditions during the growing step. In the present method, one aim of the growing step is to reduce the amount of methane producing microorganisms within the microbial biomass. Another aim may be to increase the amount and/or number of acetogens, such as homoacetogens, within the microbial biomass. During the growing step the same microbial biomass is grown under anaerobic conditions comprising carbon dioxide and hydrogen gas over a period that allows formation of an enriched biomass. The growing step may last for at least 1 week, for example at least two weeks or at least three weeks.

In some examples, the enriched biomass may comprise an increased number of acetogenic microbes and/or a reduced number of microorganisms capable of producing methane. For example, the enriched biomass may have reduced number and/or amount of methanogenic archaea. In some embodiments, the enriched microbial biomass converts less than 10 %, such as less than 5 % or less than 1 %, of the provided carbon dioxide into methane. This may be determined from the total carbon dioxide amount added to the anaerobic conditions during the producing step (CO₂ (in)). The methane and carbon dioxide content in the head-space of the reactor is measured (Methane (out) and CO₂ (out)); then the fermentation product is separated into a liquid portion and into a solid portion for example by centrifugation; the amount of short-chain fatty acids in the liquid portion is determined (total amount of short-chain fatty acids) and the amount of microbial biomass is determined from the solid portion (cell dry weight). Then, the mass balance estimations of total CO₂ added to the reactor against all products including short-chain fatty acids, microbial biomass and methane as well as residual CO₂ is performed. The following formula may be used: CO₂(in) = CO₂ (out) + Methane (out) + total amount of short-chain fatty acids + cell dry weight. Then the conversion of carbon dioxide to methane may be calculated by the following equation: (Methane (out)/CO₂ (in))* 100%. For example, in some examples less than 1 %, for example less than 0.1 % of the head space volume of a reactor comprises methane after the producing step.

In some embodiments, the carbon dioxide is used as the only carbon source for the microbial biomass and/or for the enriched microbial biomass. Thus, the growing step may be performed using a fermentation medium that does not include any additional carbon sources such as polysaccharides, glycerol, ethanol or acetate. An example of suitable fermentation medium for the growing step is given in table 1.

**Table 1. Example of fermentation medium.**

| **Fermentation medium** | | |
|---|---|---|
| **Compound** | **g/L** | **MW (g/mol)** |
| Ammonium chloride | 1 | 53,491 |
| Monopotassium phosphate | 0,33 | 1 136,086 |
| Dipotassium phosphate | 0,45 | 174,2 |
| Magnesium sulfate heptahydrate | 0,1 | 246,47 |
| Sodium bicarbonate | 10 | 84,007 |
| Cysteine hydrochloride monohydrate | 0,05 | 175,63 |
| Sodium sulfide nonahydrate | 0,05 | 240,18 |
| Wolfe's vitamin solution | 20 mL/L | |

| **Wolfe's vitamin solution** | | |
|---|---|---|
| **Compound** | **Amount (mg/L)** | |
| Biotin | 2 | |
| Folic acid | 2 | |
| Pyridoxine hydrochloride | 10 | |
| Thiamine-HCl x 2 H₂O | 5 | |
| Riboflavin | 5 | |
| Nicotinic acid | 5 | |
| D-Calcium pantothenate | 5 | |
| Vitamin B₁₂ | 2 | |
| p-Aminobenzoic acid | 5 | |
| Lipoic acid | 5 | |

In some examples, a compound inhibiting the growth of methane producing microorganisms, such as methanogens, can be added to the fermentation medium. In some embodiments, the growing step may be performed in the presence of 2-bromoethanesulfonate and/or chloroform.

The carbon dioxide and hydrogen may be provided as a gas mixture. The ratio of carbon dioxide and hydrogen in the gas mixture may be for example 30:70. The gas mixture comprises in some embodiments at least 20 % of carbon dioxide and at least 50 % of hydrogen. For example, the gas mixture may comprise 50 % of hydrogen and 50 % of carbon dioxide, or 80 % of hydrogen and 20 % of carbon dioxide. In some examples, the ratio between carbon dioxide and hydrogen in the gas mixture may range from 20:80 to 50:50. The gas mixture comprises enough carbon dioxide and hydrogen to support growth of the microbial biomass, especially growth of microorganisms comprising the enzymes of the Wood-Ljungdahl pathway.

In some embodiments, the temperature during at least some period of the growing step is below 18 °C, such as below 15 °C or below 12 °C. The at least some period may refer to the temperature being below 18 °C, such as below 15 °C or below 12 °C for at least half of the growing step. For example, the growing step may be performed at a temperature below 18 °C, such as below 15 °C or below 12 °C. In other examples, the growing step may be begun at higher temperatures, for example at 30 °C or at 25 °C, and the temperature may be gradually lowered to below 18 °C or to below 15 °C or to below 12 °C over a period of time. The period of time may be for example at least 2 days, such as at least 5 days, for example 5 to 14 days, or 10 to 20 days. Such gradual lowering of the temperature may be advantageous for the enrichment process because microorganisms in general grow better at temperatures ranging from 25 to 30 °C than at lower temperatures, and gradual lowering of the temperature to below 18 °C, such as below 15 °C may allow some microorganisms to adjust to growing at lower temperatures. For example, the growing step can be performed at a temperature of 30 °C for a first period of time and then the temperature may be gradually reduced to 18 °C or to 15 °C or to 12 °C over a second period of time. The first period of time may be for example 1 to 3 days. The second period of time may be for example 5 to 20 days. Preferably, the temperature during the growing step is at least 5 °C.

In some cases, it may be required to use compounds and/or conditions that inhibit the growth of at least some microorganisms, for example the growth of methane producing microorganisms, during the growing phase. Suitable compounds may include methanogen inhibiting agents, such as 2-bromoethanesulfonate and/or chloroform. Suitable conditions may include for example using a pH below 6.0, such as pH of 5.5. or pH of 5, during the growth phase. Such compounds and/or conditions may be especially advantageous when the temperature is gradually lowered during the growing step.

In some embodiments, the producing step is performed at a temperature below 15 °C, such as below 12 °C. The inventors have found that using a low temperature may affect the amount of different short-chain fatty acids in the final product. This may be due to changes in the microbial community and/or due to changes in activity of microorganisms within the enriched microbial biomass. It may be for example that when the microbial biomass is grown at typical temperatures of 25 to 30 °C, microorganisms producing methane are thriving and thus methane is produced. When the temperature is lowered, microorganisms producing methane may be suppressed and/or disappear from the microbial biomass and thus methane is not produced any more or the amount of produced methane is lowered. On the other hand, at temperatures below 18 °C microorganisms producing short-chain fatty acids may thrive and thus the amount of short-chain fatty acid has been observed to increase. For example, the total amount of short-chain fatty acids may range from 30 to 100 g/L of the liquid portion of the fermentation product, such as at least 40 g/L or at least 50 g/L of the liquid portion of the fermentation product. In some examples, the amount of short-chain fatty acids may reach high levels, such as at least 80 g/L of the liquid portion of the fermentation product. Liquid portion refers herein to the fermentation product from which microbial cells have been removed from for example by centrifugation. Such centrifugation may be performed for example at 6000xg for 5-10 minutes. The liquid portion of the fermentation product may refer to the supernatant obtained after removal of cell debris. Preferably, the temperature during the producing step is at least 5 °C.

In one example, microbial biomass was obtained from sludge from methane production and divided in to two samples. One was grown at 25 °C, and the second one at 15 to 22 °C. At 25 °C high level of methane was detected, but at lower temperatures only a reduced amount of methane was produced even when no methanogen inhibiting compounds, such as 2-bromoethanesulfonate or chloroform, were used.

In some embodiments, at least 4 %, such as at least 5 % or at least 10 % of the volume of the liquid portion of the fermentation product comprises at least one short-chain fatty acid. In some examples, the combined amount of short-chain fatty acids is at least 4 %, such as at least 5 % or at least 10 % of the volume of the liquid portion og the fermentation product. For example, at least 1 %, such as at least 3 %, or at least 4 % of the volume of the liquid portion of fermentation product may comprise valeric acid, isovaleric acid, hexanoic acid or combinations thereof.

In addition to the total amount of short-chain fatty acids, also the distribution of different short-chain fatty acids has been observed to change in relation to the temperature during the growing and/or producing step. Typically, short-chain fatty acids with shorter carbon chains, such as acetic acid, are produced at higher amounts than short-chain fatty acids with longer carbon chains, such as valeric acid and/or hexanoic acid. For example, when a fermentation product is produced at normal production temperatures, for example from 25 to 35 °C, most of the short-chain fatty acids within the fermentation product are acetic acid. The amount of acetic acid may be for example about 95 wt-% or 98 wt-% of all short-chain fatty acids within fermentation product produced at typical production temperatures. With the method disclosed herein, the amount of acetic acid may be reduced while the amount of other short-chain fatty acids may be increased. For example, the amount of valeric acid, isovaleric acid and/or hexanoic acid may increase when the temperature during the growing and/or producing step is lowered. In some examples, the amount of valeric acid, isovaleric acid, hexanoic acid or combinations thereof may be for example at least 1 wt-% or at least 5 wt-% or at least 10 wt-% or at least 20 wt-% of the total amount of short-chain fatty acids in the fermentation product. In some examples, the combined amount of valeric acid, isovaleric acid and hexanoic acid is at least 5 wt-% of total amount of produce short-chain fatty acids. The amount of acetic acid may be for example less than 95 wt-%, such as less than 90 wt-%, or less than 85 wt-%, from the total amount of short-chain fatty acids in the fermentation product. In some examples, the amount of valeric acid, isovaleric acid and/or hexanoic acid may increase while the amount of acetic acid may not decrease.

In some embodiments, the microbial biomass may be immobilized in a reactor before or after the growing step. The immobilization of microbial biomass may be defined as the physical confinement or localization of microorganisms to a certain region of space of a reactor without losing the desired biological activity. Immobilization may be achieved for example by using a carrier system to which the microbial biomass may be entrapped to with or without using a specific linking agent. Immobilization of microbial biomass may allow for example easier product recovery, continuous processing at industrial scale, stability and efficiency for product formation and a wider choice of reactors. Any carrier suitable for supporting biofilm growth may be utilized. Examples of suitable carrier systems include porous plastic carriers, such as K5 plastic carriers. In some examples, the carrier may be entrapped at the bottom of a reactor. In other examples, the carrier may be used in a packed bioreactor, such as in a packed-bed reactor. For a packed bioreactor examples of suitable carriers include non-woven polyethylene terephthalate (PET) fibres in different configurations.

In some embodiments, the pressure during the growing and/or the producing step may be elevated. For example, the pressure during the growing and/or the producing step may be between 1 to 12 bar. For example, the pressure during the growing and/or the producing step may be between 5 to 10 bar, or approximately 10 bar.

In some examples, a combination of immobilized microbial biomass and elevated pressure may be utilized during the growing and/or the producing step. This combination may allow better mass transfer of hydrogen gas and increased availability of hydrogen gas to the bacteria resulting in higher production rates of short-chain fatty acids. The process may further be operated at a pH below 6.0. Low pH may additionally inhibit growth of methane producing microorganisms. The conditions, such as pH, pressure and temperature, may be different during different steps of the method. Thus, at least one of the pH, temperature and pressure may differ during the growing step when compared to the producing step. In some examples, at least one of the pH, temperature and/or pressure may change during the growing step and/or the producing step. For example, the temperature may be lowered during the growing and/or producing step or the pH may be reduced during the growing and/or producing step.

In some embodiments, the producing step may be performed under anaerobic conditions comprising carbon dioxide and hydrogen gas. In these examples the producing step may be performed without the presence of any additional carbon sources for the enriched microbial biomass other than carbon dioxide. Such additional carbon sources include for example polysaccharides, glycerol, ethanol and/or acetate.

In some embodiments, the carbon dioxide may be obtained from incineration or combustion of waste material or from industrial processes, such as pulp and paper industry. Any waste materials may be used. Especially suitable waste materials include those that are difficult to recycle or use by other means. Such waste materials may comprise for example mixed waste, such as municipal waste, hazardous waste, biomass, wastewater, condensate streams, or the like. In some embodiments, the waste material comprises mixed waste or hazardous waste. In some cases, the waste material may comprise fossil fuels and/or biofuels, such as at least one fuel or mixed fuels, including support fuels, such as waste solvents and waste oils. A fuel may be selected from diesel, gasoline, kerosene, ethanol, acetone, ethyl acetate, toluene, coal, propane, methane or hydrogen. Carbon dioxide may also be obtained from different industrial processes that produce carbon dioxide as a side product. Such industries include for example the pulp and paper production, energy production or production of building materials.

The fermentation product may in some embodiments comprise at least 10 % of at least one short-chain fatty acid calculated from the weight of the fermentation product. In some examples, the enriched microbial biomass is separated from the fermentation product prior to determining the short-chain fatty acid content of the fermentation product. The enriched microbial biomass may be separated from the fermentation product with any suitable method, for example by centrifugation or filtration. In some embodiments, the fermentation product may comprise at least 20 % or at least 50 % of at least one short-chain fatty acid calculate from the weight of the fermentation product after removal of the enriched microbial biomass. In other words, the liquid portion of the fermentation product in some embodiments may comprise at least 10 %, or at least 20 % such as at least 50 % of at least one short-chain fatty acid.

In some embodiments, at least 10 %, such as at least 20 % or at least 30 % of the carbon dioxide supplied during the producing step is converted to at least one short chain fatty acid. This may be determined from the total carbon dioxide amount added to the anaerobic conditions during the producing step (CO₂ (in)). The methane and carbon dioxide content in the head-space of the reactor is measured (Methane (out) and CO₂ (out)); then the fermentation product is separated into a liquid portion and into a solid portion for example by centrifugation; the amount of short-chain fatty acids in the liquid portion is determined (total amount of short-chain fatty acids) and the amount of microbial biomass (cell dry weight) is determined from the solid portion. Then, the mass balance estimations of total CO₂ added to the reactor against all products including short-chain fatty acids, microbial biomass and methane as well as residual CO₂ is performed. The following formula may be used: CO₂(in) = CO₂ (out) + Methane (out) + total amount of short-chain fatty acids + cell dry weight. Then the conversion of carbon dioxide to short-chain fatty acids may be calculated by the following equation: (total amount of short-chain fatty acids/CO₂ (in))* 100%. In some examples, at least 5 %, such as at least 10 % of the carbon dioxide supplied during the producing step is converted to valeric acid, isovaleric acid, hexanoic acid or combinations thereof.

In other embodiments, the fermentation product may comprise at least 1 %, such as at least 10 % or at least 20 % of valeric acid, isovaleric acid and/or hexanoic acid calculated from the weight of the fermentation product. In some examples the enriched microbial biomass is separated from the fermentation product first and the amount of valeric acid, isovaleric acid and/or hexanoic acid is determined thereafter. In these examples the fermentation product may comprise at least 1 %, such as at least 10 % or at least 20 % of valeric acid, isovaleric acid and/or hexanoic acid calculated from the weight of the liquid portion of the fermentation product.

In some embodiments, the formed fermentation product is contacted with a second microbial culture to form at least one polyhydroxyalkanoate (PHA). Typically, the enriched microbial biomass is separated from the fermentation product before the fermentation product is contacted with the second microbial culture. The enriched microbial biomass may be separated for example by centrifugation or by filtration. In some examples, the fermentation product is concentrated, for example by using a suitable membrane, after the enriched microbial biomass has been separated from it but before the fermentation product is contacted with the second microbial culture.

The second microbial culture may be able to produce polyhydroxyalkanoates. A suitable second microbial culture may comprise for example at least one methylotrophic microbe. Methylotrophic microbes are microorganism that are able to use C1-compounds as an energy or carbon source for their growth and development. Methylotrophic microbes often use C1-compounds as a source of energy and as a source of carbon. Selecting certain types of organic compound(s) may have advantages in the further processing steps. For example, methanol may limit growth of many microbes and thus it can be used to minimize contaminations in the further fermentation steps. The second microbial culture may comprise for example *Cupriavidus necator*, *Methylorubrum extorquens*, *Paracoccus denitrificans* and/or *Methylobacterium organophilum.* In some examples, the second microbial culture may be a monoculture. Monoculture refers to the microbial culture comprising only a single microbial species.

In some embodiments, the at least one polyhydroxyalkanoate may be formed from the polymerization of 3-hydroxyvalerate, 3-hydroxybutyrate, 3-hydroxyhexanoate, 3-hydroxypropionate, 3-hydroxyoctanoate or combinations thereof. In some embodiments, the at least one polyhydroxyalkanoate may comprise or consist of poly(3-hydroxybutyrate-co-3-hydroxyvalerate) (PHBV), poly-3-hydroxyvalerate or combinations thereof. The at least one polyhydroxyalkanoate may comprise any combination of the above-mentioned monomers. Further, the at least one polyhydroxyalkanoate may comprise the above-mentioned monomers in any ratio. It may be advantageous if the fermentation product comprises valerate, isovalerate or hexanoic acid because these fatty acids may allow production of complex polyhydroxyalkanoates, for example PHBV. In some embodiments, the ratio between hydroxyvalerate and hydroxybutyrate in the formed at least one polyhydroxyalkanoate ranges from 1:10 to 10:1, for example from 1:5 to 5:1. In some preferred embodiments, 5 to 50 % of the monomers in the formed at least one polyhydroxyalkanoate are hydroxyvalerate monomers, such as 3-hydroxyvalerate monomers. For example, the formed at least one polyhydroxyalkanoate may comprise 3-hydroxyvalerate in the range of 10 to 40 wt%, or in the range of 15 to 30 w% calculated from the total weigh of the polyhydroxyalkanoate.

Another aspect of the present disclosure is a bioproduct obtainable by the method disclosed herein. In some embodiments, the bioproduct comprises at lest 1 % of valeric acid, isovaleric acid, and/or hexanoic acid calculated from the weight of the liquid portion of the fermentation product. For example, the bioproduct may comprise at least 5 % or 10 % or 20 % of valeric acid, isovaleric acid, and/or hexanoic acid calculated from the weight of the liquid portion of the fermentation product.

### Example

Microbial biomass was gathered from methane producing lab reactors, all together from 8 different sources. The samples were mixed together to form one sludge. The sludge was pre-treated by incubating the sludge with small amount of water in anaerobic conditions to exhaust/use up all carbon sources within the sludge. In the small-scale experiments, 100 mL serum bottles containing 25 mL of medium (as disclosed in Table 1) was inoculated with a pretreated sludge. Methanogen inhibiting agents (2 mmol/L 2-bromoethanesulfonate (BES) and or 0,05 v/v chloroform) were added to some of the bottles. The bottles were flushed with nitrogen gas to establish anaerobic conditions, after which carbon dioxide and hydrogen gas mixture (30 % and 70 %) was fed to the bottles on a daily basis. Apart from the gas mixture, no additional carbon sources was provided for the cultures. The incubation of the serum bottles is done a.) in an incubator at 25 °C with 200 rpm shaking b.) on laboratory bench with fluctuation temperature between 15-22 °C c.) on laboratory bench at 13-16 °C d.) in a water bath at 15 °C.

The cultures were monitored weekly with offline analysis, including pH measurement, organic acid analysis and gas chromatography for qualitative methane detection. The cultures were harvested once the organic acid content reached 1500-3000 mg/L, after which the cells were separated and transferred to a serum bottle with fresh media (as shown in table 1) and enrichment process of the biomass was continued.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

## Claims

1. A method comprising the steps of
- providing a microbial biomass comprising at least one microorganism expressing the enzymes of the Wood-Ljungdahl pathway,
- growing the microbial biomass under anaerobic conditions comprising carbon dioxide and hydrogen gas to form an enriched microbial biomass,
- producing a fermentation product comprising at least one short-chain fatty acid by using the enriched microbial biomass,
wherein the producing step is performed at a temperature below 18°C.

2. The method according to claim 1, wherein the temperature during the producing step is below 15°C, such as below 12 °C.

3. The method according to claim 1 or 2, wherein the temperature during at least some period of the growing step is below 18 °C, such as below 15 °C.

4. The method according to any of the preceding claims, wherein the fermentation product comprises at least 40 g/L of short-chain fatty acids calculated from a liquid portion of the fermentation product.

5. The method according to any of the preceding claims, wherein the short-chain fatty acid is selected from acetic acid, propionic acid, butyric acid, isobutyric acid, isovaleric acid, valeric acid, hexanoic acid or combinations thereof.

6. The method according to any of the preceding claims, wherein the producing step is performed under anaerobic conditions comprising carbon dioxide and hydrogen gas.

7. The method according to any of the preceding claims, wherein the pressure during the growing step and/or the producing step is between 1 to 12 bar.

8. The method according to any of the preceding claims, wherein the pH during the growing step and/or the producing step is below 6.0.

9. The method according to any of the preceding claims, wherein the microbial biomass is obtained from a sludge, for example from sludge from methane production or waste water treatment.

10. The method according to any of the preceding claims, wherein the carbon dioxide is used as the only carbon source for the microbial biomass and/or for the enriched microbial biomass.

11. The method according to any of the preceding claims, wherein the fermentation product comprises at least 1 %, such as at least 5 %, of valeric acid, isovaleric acid, hexanoic acid or combinations thereof of the total amount of produced short-chain fatty acids.

12. The method according to any of the preceding claims, wherein the fermentation product is contacted with a second microbial culture to form at least one polyhydroxyalkanoate.

13. The method according to claim 12, wherein the at least one polyhydroxyalkanoate is formed from the polymerization of 3-hydroxyvalerate, 3-hydroxybutyrate, 3-hydroxyhexanoate, 3-hydroxypropionate, 3-hydroxyoctanoate or combinations thereof.

14. A fermentation product obtainable by the method according to any of the preceding claims.

15. The fermentation product according to claim 14 comprising at least 1 %, such as at least 5 %, of valeric acid, isovaleric acid, and/or hexanoic acid calculated from the weight of the liquid portion of the fermentation product.
